# EUROPÄISCHE PATENTSCHRIFT

(11) **EP 0 295 551 B1**
(45) Veröffentlichungstag und Bekanntmachung des Hinweises auf die Patenterteilung: **14.04.1993**
(21) Anmeldenummer: 88109088.0
(22) Anmeldetag: 08.06.1988
(51) Int. Cl.: C07C 67/333, C07C 67/38, C07C 67/39, C07C 69/44, C07C 55/14

(54) **Verfahren zur Herstellung von Adipinsäure**
Method for the production of adipic acid
Procédé pour la fabrication de l'acide adipique

(30) Priorität: 15.06.1987 DE 3719936
(43) Veröffentlichungstag der Anmeldung: 21.12.1988
(73) Patentinhaber: BASF Aktiengesellschaft, 67063 Ludwigshafen (DE)
(72) Erfinder: Kummer, Rudolf, Dr., D-6710 Frankenthal (DE); Merger, Franz, Dr., D-6710 Frankenthal (DE); Bertleff, Werner, Dr., D-6806 Viernheim (DE); Fischer, Rolf, Dr., D-6900 Heidelberg (DE)

(56) Entgegenhaltungen:
- EP-A- 0 125 567
- EP-A- 0 131 860
- US-A- 3 941 851

## Beschreibung

Bei der Herstellung von Adipinsäure durch Hydroesterifizierung von Pentensäureestern erhält man erhebliche Mengen an 2-Methylglutarsäureestern und 3-Ethylbernsteinsäureestern als Nebenprodukt. Somit erhält man Adipinsäure lediglich nur aus einem Bruchteil der eingesetzten Pentensäureester.

Aus der europäischen Patentschrift 131 860 ist bereits bekannt, daß man Pentensäureester in Gegenwart von Kobalt- oder Rhodiumcarbonylkomplexen hydroformyliert und die so erhaltenen 5-Formylvaleriansäureester mit molekularem Sauerstoff zu Adipinsäuremonoester oxidiert. Auch nach diesem Verfahren beträgt die Selektivität zu 5-Formylvaleriansäureestern 88,1 % und die Ausbente 71,8 %, so daß erhebliche Mengen an Nebenprodukten in Kauf genommen werden müssen.

Es wurde auch schon versucht, zunächst 3-Pentensäureester katalytisch zu 4-Pentensäureestern zu isomerisieren und diese anschließend zu Formylvaleriansäureestern zu hydroformylieren, wie aus der europäischen Patentschrift 125 567 bekannt ist. Auch nach diesem Verfahren gelingt es nicht, den Anteil an isomeren Formylvaleriansäureestern auf ein technisch vertretbares Maß zu reduzieren.

Ferner ist aus der US-Patentschrift 4,517,400 bekannt, daß man Aldehyde in Gegenwart von Zeolithen, die katalytisch aktive Metalle enthalten, in die entsprechenden Ausgangsolefine dehydrocarbonyliert. Beim Erhitzen eines Gemisches aus n- und i-Butyraldehyd auf eine Temperatur von 300°C in Gegenwart solcher Katalysatoren wird jedoch nur n-Butyraldehyd zu Propylen decarbonyliert während i-Butyraldehyd nicht angegriffen wird. Eine Übertragung dieser Reaktion auf Formylvaleriansäureester erschien auch nicht angezeigt, da Formylbuttersäureester ausweislich der europäischen Patentanmeldung 81 090 unter Cyclisierung Dihydropyrone ergeben und eine analoge Cyclisierung der isomeren Formylvaleriansäureester zu erwarten war.

Es war deshalb die technische Aufgabe gestellt, ein Verfahren zur Herstellung von Adipinsäure, ausgehend von Pentensäureestern zur Verfügung zu stellen, bei dem Pentensäureester möglichst vollständig in Adipinsäure überführt wird, eine zusätzliche Isomerisierung von Pentensäureestern zu 4-Pentensäureester nicht erforderlich ist und schließlich der Anfall an nicht verwertbaren verzweigten Isomeren und weiteren Nebenprodukten vermindert wird.

Diese Aufgabe wird gelöst in einem Verfahren zur Herstellung von Adipinsäure, welches folgende Schritte umfaßt:
a) Hydroformylierung von Pentensäureestern durch Umsetzung mit Kohlenmonoxid und Wasserstoff bei einer Temperatur von 60 bis 160°C und unter erhöhtem Druck in Gegenwart von Kobalt- oder Rhodiumcarbonylkomplexen unter Bildung eines Gemisches aus 5-, 4- und 3-Formylvaleriansäureestern.
b) Abtrennen von 5-Formylvaleriansäureestern aus dem so erhaltenen Gemisch aus 5-, 4- und 3-Formylvaleriansäureestern unter Verbleib eines im wesentlichen aus 4- und 3-Formylvaleriansäureestern bestehenden Gemisches
c) Dehydrocarbonylieren des im wesentlichen aus 4- und 3-Formylvaleriansäureestern bestehenden Gemisches in Gegenwart von mindestens einem Element der 8. Nebengruppe des Periodischen Systems bei einer Temperatur von 50 bis 400°C unter Bildung von Pentensäureestern und deren Rückführung in Stufe a) zur Hydroformylierung
d) Oxidieren der 5-Formylvaleriansäureester aus Stufe b) mit molekularem Sauerstoff oder solchen enthaltenden Gasen zu Adipinsäuremonoestern und
e) Hydrolysieren der Adipinsäuremonoester zu Adipinsäure.

Das neue Verfahren hat den Vorteil, daß man ausgehend von Pentensäureestern Adipinsäure erhält, unter Vermeidung von unerwünschten Isomeren. Weiter hat das neue Verfahren den Vorteil, daß der Anfall an anderen Nebenprodukten und cyclischen Verbindungen minimiert wird.

In der Stufe a) werden Pentensäureester hydroformyliert. Geeignete Pentensäureester leiten sich von Alkanolen mit 1 bis 12 Kohlenstoffatomen oder Cycloalkanolen mit 5 bis 8 Kohlenstoffatomen ab. Besonders bevorzugt sind Pentensäure-C₁ -C₁ ₂-alkylester, insbesondere Pentensäure-C₁-C4-alkylester, z.B. Pentensäuremethylester. Geeignete Verbindungen sind z.B. 4-Pentensäureester, 3-Pentensäureester und 2-Pentensäureester einzeln oder Gemische derselben. Beispielsweise seien genannt Methyl-, Ethyl-, Propyl-, Isopropyl-, Butyl-, Hexyl-, Nonyl-, Dodecyl-, Cyclopentyl- oder Cyclohexylester der 2-, 3- oder 4-Pentensäure.

Die Hydroformylierung der Pentensäureester wird bei einer Temperatur von 60 bis 160°C, insbesondere von 80 bis 120°C, durchgeführt. Hierbei hält man erhöhten Druck, vorteilhaft von 5 bis 300 bar, ein. Die Hydroformylierung der Pentensäureester erfolgt durch Umsetzen mit Kohlenmonoxid und Wasserstoff. In der Regel enthält das Gasgemisch Kohlenmonoxid und Wasserstoff im Molverhältnis von 1:0,5 bis 1:10, insbesondere von 1:1 bis 1:2.

Die Hydroformylierung wird in Gegenwart von Kobalt- oder Rhodiumcarbonylkomplexen durchgeführt. Die Carbonylkomplexe können vor der Reaktion aus Kobalt- oder Rhodiumsalzen durch Umsetzen mit Kohlenmonoxid hergestellt werden. Vorteilhaft werden sie in situ aus den Salzen des Rhodiums oder Kobalts gebildet. Vorzugsweise sind die verwendeten Kobalt- oder Rhodiumcarbonylkomplexe zusätzlich durch tertiäre Phosphine oder tertiäre Phosphite modifiziert. Geeignete Phosphine oder Phosphite haben Alkylreste mit bis zu 12 Kohlenstoffatomen und/oder Phenylreste die zusätzlich Alkylgruppen mit bis zu 4 Kohlenstoffatomen als Substituenten enthalten können. Bevorzugt verwendet man Triphenylphosphin, substituierte Triarylphosphine wie Tritolylphosphin, ferner Alkyldiarylphosphine wie Hexyldiphenylphosphin.

Sofern man Kobaltcarbonylkomplexe verwendet, hat es sich bewährt, wenn man 0,01 bis 1 Mol%, vorzugsweise 0,05 bis 0,3 Mol%, insbesondere 0,08 bis 0,25 Mol%, Kobaltcarbonylkomplexe, berechnet als Kobalt, bezogen auf eingesetzte Pentensäureester anwendet. Vorteilhaft hält man zudem einen Umsatz der eingesetzten Pentensäureester von 10 bis 50 %, insbesondere 20 bis 40 % ein. Hierdurch wird die Bildung von Nebenprodukten durch Hydrieren und Aldolisieren vermindert. Unter diesen Bedingungen ist es möglich, auf die Mitverwendung von Lösungsmitteln zu verzichten und Kobaltcarbonylkomplexe, die einen Gehalt von bis zu 20 Mol je Mol Kobalt an tertiären Stickstoffbasen enthalten, zu verwenden ohne daß die Hydroformylierung negativ beeinflußt wird. Solche Katalysatoren fallen z.B. bei der Hydroesterifizierung von Butadien zu Pentensäureester an, wie sie in der europäischen Patentschrift 31 100 beschrieben wird.

Sofern Rhodiumcarbonylkomplexe verwendet werden, hält man vorteilhaft eine Temperatur von 100 bis 120°C und einen Druck von 5 bis 20 bar ein. Die Konzentration an Rhodiumcarbonylkomplexen beträgt zweckmäßig 5 bis 500 ppm, berechnet als Metall und bezogen auf das Reaktionsgemisch. Die Modifizierung der Rhodiumcarbonylkomplexe mit den vorgenannten Phosphiten oder Phosphiten hat sich besonders bewährt. Vorteilhaft wendet man Phosphine oder Phosphite mit 3 bis 100fachem molarem Überschuß, bezogen auf Rhodium, an.

Es ist auch möglich, die Hydroformylierung in Gegenwart von unter Reaktionsbedingungen inerten Lösungsmitteln durchzuführen. Geeignete Lösungsmittel sind beispielsweise Ether wie Tetrahydrofuran, ferner Carbonsäureester wie Valeriansäureester, Buttersäureester oder Essigsäureester sowie Kohlenwasserstoffe wie Toluol.

Zweckmäßigerweise wird das Hydroformylierungsgemisch nach dem Entspannen nach bekannten Methoden aufgearbeitet. Eine geeignete Methode für die Aufarbeitung von Kobaltcarbonylkomplexe enthaltenden Reaktionsgemischen ist beispielsweise in der europäischen Patentschrift 31 100 beschrieben. Hierbei wird der Hydroformylierungsaustrag nach dem Entspannen mit Oxidationsmitteln wie Wasserstoffperioxid oder molekularen Sauerstoff enthaltenden Gasen, insbesondere Luft, vorteilhaft in einer Menge von 2 bis 10 Oxidationsäquivalenten je Mol Kobaltkatalysator in Gegenwart einer wäßrigsauren Lösung, z.B. wäßrige Ameisensäure oder Essigsäure, Buttersäure, Valeriansäure oder 2-Ethylhexansäure bei einer Temperatur z.B. von 80 bis 160°C, insbesondere 100 bis 130°C, behandelt. Je nach dem Grad der Durchmischung ist die Abtrennung des Kobaltkatalysators bereits nach wenigen Sekunden oder Bruchteilen von Sekunden beendet. Die Kobalt enthaltende wäßrige Phase wird zweckmäßig durch Dekantieren abgetrennt. Man erhält als organische Phase ein Gemisch aus 5-, 4- und 3-Formylvaleriansäureestern, das als Nebenprodukte noch Hochsieder und Valeriansäureester sowie nicht umgesetzte Pentensäureester sowie ggf. Lösungsmittel enthält.

Das vom Katalysator befreite Gemisch von Formylvaleriansäureestern wird durch Destillation aufgetrennt. In der Regel trennt man zunächst gegebenenfalls mitverwendetes Lösungsmittel und nicht umgesetzte Pentensäureester einzeln oder als Gemisch ab und führt diese zweckmäßig wieder in die Hydroformylierungsstufe zurück. Aus dem so erhaltenen 5-, 4- und 3-Formylvaleriansäureester und gegebenenfalls geringe Mengen Schwersieder enthaltenden Gemisch wird in der Stufe b) 5-Formylvaleriansäureester durch Destillation abgetrennt, unter Verbleib eines Gemisches das im wesentlichen aus 4- und 3-Formylvaleriansäureestern besteht. Je nach Wirksamkeit der Trennung können auch noch geringe Mengen, z.B. bis zu 5 Gew.% 5-Formylvaleriansäureester enthalten sein.

In der Stufe c) wird dann das so erhaltene Gemisch, das im wesentlichen aus 4- und 3-Formylvaleriansäureestern besteht, unter Bildung von Pentensäureestern bei einer Temperatur von 50 bis 400°C in Gegenwart von mindestens einem Element der 8. Nebengruppe des Periodischen Systems dehydrocarbonyliert und die erhaltenen Pentensäureester in die Stufe a) zur Hydroformylierung zurückgeführt.

Für das erfindungsgemäße Verfahren können zwar die reinen 4- und 3-Formylvaleriansäureester verwendet werden, im allgemeinen verwendet man jedoch zweckmäßig deren Gemische, die in Abhängigkeit vom Wirkungsgrad der Destillation auch noch 5-Formylvaleriansäureester enthalten können. Ein typisches Gemisch enthält beispielsweise 60 bis 75 Gew.% 4-Formylvaleriansäureester, 25 bis 35 Gew.% 3-Formylvaleriansäureester sowie bis zu 5 Gew.% 5-Formylvaleriansäureester. Als Dehydrocarbonylierungsprodukte erhält man 4-, 3- und 2-Pentensäureester, vorwiegend 3-Pentensäureester.

Geeignete homogene Katalysatoren sind Komplexverbindungen von Edelmetallen der 8. Nebengruppe des Periodischen Systems, insbesondere von Ruthenium oder Rhodium. Besonders geeignet sind Halogene, wie Chlor oder Brom und Phosphine oder Phosphite enthaltende Ruthenium- oder Rhodiumkomplexe die zusätzlich als Liganden Kohlenmonoxid enthalten können. Besonders bevorzugt werden als Modifizierungsmittel tertiäre organische Phosphine oder Phosphite verwendet. Solche Phosphine oder Phosphite haben bevorzugt als Substituenten Alkylreste mit bis zu 18 Kohlenstoffatomen, Cycloalkylreste mit 5 bis 12 Kohlenstoffatomen, Aralkylreste mit 7 bis 10 Kohlenstoffatomen oder Arylreste mit 6 bis 10 Kohlenstoffatomen, insbesondere Phenylreste. Die Reste können gleich oder verschieden sein. Beispiele für geeignete Komplexverbindungen sind RhCl[P(C₆H₅)₃]₃, Ru₂Cl₃[P(C₆H₅)(C₂H₅)₂)₆]Cl, RhBr(CO)[P(C₆H₅)₃]₂, HRuCl(CO)[P(C₆H₅)₂]₃, RhCl(CO)[P(C₆H₅)₃]₂.

Bevorzugt werden Trägerkatalysatoren verwendet, die mindestens eines der Elemente der 8. Nebengruppe des Periodischen Systems wie Palladium, Platin, Ruthenium, Rhodium, Osmium, Iridium, Eisen, Kobalt oder Nickel, insbesondere jedoch Edelmetalle dieser Gruppe enthalten. Vorteilhaft sind auch Trägerkatalysatoren, die mindestens zwei Edelmetalle der 8. Nebengruppe des Periodischen Systems wie Ruthenium, Rhodium, Palladium oder Platin enthalten. Andere bevorzugte Katalysatoren enthalten mindestens ein Metall der vorgenannten Edelmetalle der 8. Nebengruppe des Periodischen Systems und zusätzlich mindestens ein Metall ausgewählt aus der gruppe Eisen, Kobalt und Nickel.

Die Trägerkatalysatoren haben vorteilhaft einen Gehalt an aktiven Metallen der 8. Nebengruppe des Periodischen Systems von 0,01 bis 10, vorzugsweise 0,05 bis 5, insbesondere 0,05 bis 1 Gew.%, bezogen auf die Summe aus Träger und katalytisch aktiven Metallen, berechnet als Metalle. Als Träger werden vorteilhaft Aluminiumoxid, Siliciumdioxid, Titandioxid, Zirkondioxid, Zinkoxid, Lanthanoxid, Bariumsulfat oder Gemische dieser Oxide sowie Aluminiumsilikate verwendet.

Besonders vorteilhaft enthalten die genannten Trägerkatalysatoren noch mindestens ein Element der 1. bis 7. Nebengruppe des Periodischen Systems und/oder Elemente der Seltenen Erden wie Zink, Kupfer, Silber, Lanthan, Titan, Vanadium, Chrom, Molybdän, Wolfram, Mangan, Rhenium, Cer, Neodym oder Praseodym, vorteilhaft in einer Menge von 0,05 bis 2 Gew.%, bezogen auf das Gesamtgewicht des Katalysators (Träger und katalytisch aktive Metalle), berechnet als Metall.

Bewährt haben sich z.B. Tränkkatalysatoren, bei denen die katalytisch wirksamen Metalle an der Oberfläche des Trägers angereichert sind. Derartige Katalysatoren werden in an sich bekannter Weise durch Tränken vergeformter Träger wie Pellets, Kugeln oder Stränge mit einer wäßrigen Lösung der Metallsalze, die beim Erhitzen in ihre Oxide übergehen, z.B. der Nitrate erhalten, die dann getrocknet, calciniert und direkt oder gegebenenfalls nach Reduktion mit Wasserstoff oder anderen Reduktionsmitteln eingesetzt werden können.

Die in der Stufe c) verwendeten Katalysatoren zeigen über einen längeren Zeitraum hohe Aktivität. Verbrauchte Katalysatoren lassen sich durch Behandeln mit Sauerstoff enthaltenden Gasen, z.B. Luft, bei einer Temperatur von 350 bis 500°C und gegebenenfalls anschließende Reduktion regenerieren.

Bei der Dehydrocarbonylierung in Stufe c) hält man vorteilhaft eine Temperatur von 60 bis 350°C, insbesondere 100 bis 280°C, vorzugsweise von 120 bis 200°C ein. Im allgemeinen wird die Spaltung unter Atmosphärendruck durchgeführt. Es ist jedoch auch möglich, verminderten Druck oder erhöhten Druck, zweckmäßig im Bereich von 10 mbar bis 20 bar, anzuwenden. Im allgemeinen hält man eine Katalysatorbelastung von 0,01 bis 40, vorzugsweise 0,1 bis 20 kg Formylvaleriansäureester je kg Katalysator und Stunde ein.

Vorteilhaft führt man die Dehydrocarbonylierung in Stufe c) unter Mitverwendung von molekularem Sauerstoff oder molekularen Sauerstoff enthaltenden Gasen, die neben Sauerstoff Inertgase wie Stickstoff, Kohlendioxid, Argon oder Wasserdampf enthalten, z.B. Luft, durch. Vorzugsweise wendet man ein Molverhältnis von Formylvaleriansäureestern zu molekularem Sauerstoff von 1:0,05 bis 1:3, insbesondere von 1:0,2 bis 1:1,5, z.B. von 1:0,25 bis 1,25 an. Hierdurch wird die Lebensdauer des Katalysators erhöht und insbesondere die Ausbeute an Pentensäureestern gesteigert. Die Mitverwendung von molekularem Sauerstoff war nicht angezeigt, da 5-Formylvaleriansäuremethylester schon bei 50°C durch molekularen Sauerstoff mit 96 % der Ausbeute zu Adipinsäuremonomethylester oxidiert wird, wie aus der eingangs erwähnten EP Patentschrift 131 860 bekannt ist und somit zu erwarten war, daß 4- und 3-Formylvaleriansäureester analog zu 2-Methylglutarsäure- und 3-Ethylbernsteinsäuremonoester oxidiert werden.

Es kann zweckmäßig sein, die Dehydrocarbonylierung der Formylvaleriansäureester in Stufe c) unter zusätzlichem Mitverwenden von Verdünnungsmitteln durchzuführen. Geeignete Verdünnungsmittel sind z.B. Wasser, Alkohole wie Methanol, Ethanol, Butanol oder Cyclohexanol ferner Ether, wie Dioxan oder Tetrahydrofuran sowie Chlorkohlenwasserstoffe, wie Methylenchlorid, Chloroform oder 1,2-Dichlormethan. Weiterhin aliphatische, cycloaliphatische oder aromatische Kohlenwasserstoffe, wie Benzol, Toluol, Cyclohexan oder Paraffine, Darüber hinaus Ester wie Essigsäureester oder Propionsäureester. Vorteilhaft wird der Alkohol verwendet, der dem in den Formylvaleriansäureestern gebundenen Alkohol entspricht. Edukt und Produkt sind so durch eine ausreichende Siedepunktdifferenz destillativ gut trennbar. Es hat sich bewährt, wenn das Molverhältnis von Formylvaleriansäureestern zu Verdünnungsmitteln von 1:0,1 bis 1:50, insbesondere von 1:0,5 bis 1:20 beträgt. Besonders bevorzugte Verdünnungsmittel sind Wasser und Alkanole mit 1 bis 6 Kohlenstoffatomen, insbesondere Methanol.

Die Dehydrocarbonylierung in der Stufe c) kann diskontinuierlich oder kontinuierlich als Festbettreaktion mit Festbettkatalysatoren, beispielsweise in Sumpf- oder Rieselphase in der Flüssig- oder Gasphase oder als Wirbelbettreaktion mit in auf- und abwirbelnder Bewegung befindlichen Katalysatoren in der Gasphase, ferner in der Flüssigphase mit löslichen Katalysatoren oder suspendierten Trägerkatalysatoren durchgeführt werden.

Eine bevorzugte Ausführungsform in der Stufe c) in Flüssigphase führt man beispielsweise durch, indem man Formylvaleriansäureester und gegebenenfalls Verdünnungsmittel mit Sauerstoff enthaltenden Gasen bei einer Temperatur unterhalb des Siedepunkts des Formylvaleriansäureesters über einen festen Katalysator leitet oder in Gegenwart eines suspendierten festen Katalysators oder eines gelösten homogenen Katalysators erhitzt. Das erhaltene flüssige Reaktionsprodukt wird anschließend nach Abtrennung der Katalysatoren durch Destillation in Pentensäureester sowie gegebenenfalls Verdünnungsmittel und nicht umgesetzte Formylvaleriansäureester getrennt.

Eine andere bevorzugte Ausführungsform der Stufe c) in der Gasphase führt man beispielsweise durch, indem man ein Gemisch aus Formylvaleriansäureester und gegebenenfalls Verdünnungsmittel verdampft und es dann zusammen mit Luft, zweckmäßig mit zusätzlichem Trägergas, wie Stickstoff-Kohlendioxid oder Argon bei den angegebenen Temperaturen gasförmig in eine fest angeordnete oder eine in auf- und abwirbelnde Bewegung befindliche Katalysatorschicht einleitet. Der Reaktionsaustrag wird kondensiert und anschließend durch fraktionierende Destillation aufgetrennt. Nicht umgesetzte Formylvaleriansäureester werden zweckmäßig in die Stufe c) zurückgeführt. Das erhaltene Gemisch aus 4-, 3- und 2-Pentensäureestern wird wiederum in die Hydroformylierungsstufe a), gegebenenfalls mit Pentensäureestern die in Stufe b) anfallen zurückgeführt. Die als Nebenprodukte anfallenden Valeriansäureester können ausgeschleust oder ebenfalls als Lösungsmittel in die Stufe a) zurückgeführt werden.

In der Stufe d) werden die in Stufe b) erhaltenen 5-Formylvaleriansäureester mit molekularem Sauerstoff oder solchen enthaltenden Gasen zu Adipinsäuremonoestern oxidiert. Die Oxidation führt man vorteilhaft bei einer Temperatur von 20 bis 100°C, insbesondere 50 bis 97°C und unter einem Druck von 1 bis 10 bar durch. Die molekularen Sauerstoff enthaltenden Gase können z.B. bis zu 80 Vol.% Inerte, wie Stickstoff, Kohlendioxid oder Edelgase enthalten. Die Oxidation läuft in der Regel ohne Katalysator ab. Sie kann aber auch durch Zusatz von Katalysatoren wie Alkalihydroxide, z.B. Kaliumhydroxid oder Natriumhydroxid in Mengen von 0,001 bis 0,5 Gew.% oder Metallsalzen von Cobalt oder Mangan, z.B. Cobaltacetat oder Manganacetat in Mengen von 0,001 bis 0,1 Gew.%, vorzugsweise 0,02 bis 0,08% Gew.%, berechnet als Metall zusätzlich beschleunigt werden. Zweckmäßig erhält man aus dem Reaktionsgemisch reine Adipinsäuremonoester durch Destillation.

In der Stufe e) werden die so erhaltenen Adipinsäuremonoester zu Adipinsäure hydrolysiert. Vorteilhaft wendet man je Mol Adipinsäuremonoester 1 bis 200 Mol, insbesondere 50 bis 150 Mol Wasser an. Darüber hinaus können unter Reaktionsbedingungen inerte Lösungsmittel mitverwendet werden. Geeignete Lösungsmittel sind beispielsweise Kohlenwasserstoffe wie Cyclohexan oder Toluol, weiterhin Halogenkohlenwasserstoffe wie Dichlormethan oder Tetrachlormethan, ferner Ether wie Dioxan oder Diglycine. Falls Lösungsmittel mitverwendet werden, setzt man die Adipinsäuremonoester als 1 bis 90 gew.%ige Lösung, insbesondere 5 bis 20 gew.%ige Lösung, vorteilhaft als wäßrige Lösung ein.

Die Hydrolyse wird zweckmäßig bei einer Temperatur von 30 bis 200°C durchgeführt. Vorteilhaft wendet man eine Temperatur von 50 bis 120°C an. Im allgemeinen hält man Atmosphärendruck ein. Es ist jedoch auch möglich, schwach verminderten Druck oder schwach erhöhten Druck, z.B. bis zu 20 bar anzuwenden.

Zweckmäßig wird die Hydrolyse unter Mitverwendung von sauren Mitteln durchgeführt. Geeignete saure Mittel sind beispielsweise Sulfonsäuren, Lewissäuren, nicht oxidierende Mineralsäuren, niedere Fettsäuren oder stark saure Kationenaustauscher. Geeignete saure Mittel sind beispielsweise nicht oxidierende Mineralsäuren, wie Schwefelsäure, Chlorwasserstoffsäure, Bromwasserstoffsäure, weiterhin Sulfonsäure, wie p-Toluolsulfosäure oder Lewissäuren, wie Zinkchlorid, darüber hinaus niedere aliphatische Carbonsäuren wie Ameisensäure, Essigsäure oder Propionsäure und Oxalsäure sowie stark saure Ionenaustauscher, die z.B. aufgebaut sind aus vernetztem Polystyrol mit Sulfonsäuregruppen oder Phenolharze mit Sulfongruppen, ferner saure Zeolithe.

Vorteilhaft wendet man Säuren, zur Hydrolyse in homogener Phase in katalytischen Mengen, z.B. von 0,002 bis 0,25 Mol/Mol Adipinsäuremonoester an. Aliphatische Carbonsäuren werden im allgemeinen in Mengen von 0,1 bis 1 Mol/Mol Adipinsäuremonoester eingesetzt.
Besonders bevorzugt werden stark saure Kationenaustauscher angewandt.

Das Verfahren kann chargenweise oder vorteilhaft kontinuierlich, z.B. in einer Rührkesselkaskade durchgeführt werden. Hierbei ist es zweckmäßig, den bei der Hydrolyse anfallenden Alkohol fortlaufend durch Destillation aus dem Reaktionsgemisch abzutrennen. Bei der Verwendung von stark sauren Kationenaustauschern ist es vorteilhaft, die Umsetzung so durchzuführen, daß man die stark sauren Kationenaustauscher, z.B. in einem Rohrreaktor fest anordnet und das Reaktionsgemisch in Rieselfahrweise darüber leitet. In einer besonders vorteilhaften Ausführungsform wird das Reaktionsgemisch zunächst in Rieselfahrweise durch eine erste Reaktionszone mit fest angeordneten stark sauren Kationenaustauschern geleitet und dann in einer zweiten Reaktionszone über fest angeordnete stark saure Kationenaustauscher im Kreis geführt, und das Reaktionsgemisch in dem Maße entommen, wie es der ersten Zone zugeführt wird.

Nach einer anderen vorteilhaften Arbeitsweise lietet man Adipinsäuremonoester und Wasser im Überschuß durch eine mit stark sauren Kationenaustauschern beschickte Kolonne, destilliert am oberen Ende die Alkohole ab und übernimmt am anderen Ende eine wäßrige Lösung, von Adipinsäure. Aus der so erhaltenen wäßrigen Lösung läßt sich Adipinsäure durch Kristallisation in reiner Form gewinnen.

Adipinsäure, die nach dem erfindungsgemäßen Verfahren erhältlich ist, ist ein wichtiger Ausgangsstoff zur Herstellung von Polyamiden.

Das Verfahren nach der Erfindung sei am folgendem Beispiel veranschaulicht.

### Beispiel

a) Die Hydroformylierungsversuche wurden in einer kontinuierlich betriebenen Apparatur durchgeführt. Diese besaß eine Flüssigdosierpumpe, mit der Pentensäuremethylester und der gelöste Cobaltkatalysator in die 2 hintereinander geschalteten Rührautoklaven gefördert wurden. Das Synthesegas wurde druckgeregelt mit dem flüssigen Zulauf vor dem ersten Reaktor zusammengeführt. Die beiden Reaktoren besaßen Flüssigvolumina von 1,2 bzw. 1,12 l. Der zweiphasige Austrag wurde unter Druck in einem Behälter gesammelt, aus dem eine bestimmte Abgasmenge abgeregelt wurde. Die Flüssigphase wurde standgeregelt in ein Vorlagegefäß entspannt.
   In dieser Apparatur wurde eine Mischung von 3-Pentensäuremethylester mit 800 ppm Cobalt (als Co₂(CO)₈ (360 ml/h) bei 100°C und 130 bar (CO/H₂ = 1) hydroformyliert. Es wurde ein Austrag von 327 g/h erhalten, der sich wie folgt zusammensetzt:
   38,1 % (m/m) Formylvaleriansäuremethylester (mit 69,6 % n-Anteil)
   0,4 % (m/m) Valeriansäuremethylester
   61,2 % (m,m) Pentensäuremethylester
   Dieses Ergebnis entspricht einem Umsatz von 33 %, einer Selektivität an gesamtem Formylvalerianester von 98,8 % und einer Selektivität an 5-Formylvalerianester von 68,8 %.
   Dieser Austrag wurde mit stündlich 150 ml 5 %iger Essigsäure unter Einleiten von 5 l/h Luft unter guter Durchmischung durch ein Rohr geleitet. Nach der Phasentrennung wurden 154 ml einer 0,2 %igen Cobaltacetatlösung (ber. Cobalt) abgetrennt.
b) In einer diskontinuierlichen Destillation wurden 835 g der organischen Phase in ca. 3 g Valeriansäuremethylester, 495 g 3-Pentensäuremethylester, 12 g 2-trans-Pentensäuremethylester sowie ca. 315 g eines 5-, 4-, 3-Formylvaleriansäuremethylester-Gemisches und 6 g Rückstand getrennt.
   Das Formylvaleriansäuremethylester-Gemisch wurde durch weitere fraktionierende Destillation in 215 g 5-Formylvaleriansäuremethylester (99 %ig) 90 g eines Gemisches aus 5-, 4- und 3-Formylvaleriansäuremethylester (2 %, 5-, 70 % 4- und 28 % 3-FVSE) und 10 g Rückstand aufgetrennt.
c) Innerhalb einer Stunde wurde das Gemisch aus 90 g 5-, 4- und 3-Formylvaleriansäuremethylester aus Stufe b) zusammen mit 180 g Methanol in einen Verdampfer gepumpt und von dort zusammen mit 70 l Luft bei 250°C über 200 g eines SiO₂-Trägerkatalysators geleitet, der 0,5 Gew.% Ru, 0,5 Gew.% Rh und 0,5 Gew.% Pt enthielt. Bei der destillativen Aufarbeitung der kondensierten Reaktionsausträge (260 g) wurden nach Abtrennung von Methanol und Wasser 53 g Pentenester und Valerianester (15 % 4-, 53 % 3- und 15 % 2-trans-Pentensäuremethylester, 17 % Valeriansäuremethylester) erhalten. Außerdem wurden 10 g eines Gemisches aus 4- und 3-Formylvaleriansäuremethylester zurückgewonnen. Das Gemisch aus 4-, 3-, 2-Pentensäuremethylester wurde in Gegenwart des Valeriansäuremethylesters in Stufe a) wiederum mit praktisch gleicher Ausbeute wie ausgehend von 3-Pentensäuremethylester zu Formylvaleriansäureestern hydroformyliert.
d) 215 g von dem in Stufe b) anfallenden 5-Formylvaleriansäuremethylester wurden in eine Blasensäule gefüllt und 5,5 Stunden lang bei 55 ± 2°C und Normaldruck mit 8 l Sauerstoff pro Stunde begast. Nach dieser Reaktionszeit war der Umsatz quantitativ. Durch fraktionierende Destillation wurden aus dem Oxidat 225 g Adipinsäuremonomethylester von Siedepunkt 113°C/0,6 mbar erhalten (Ausbeute 95 %).
e) In einer Lösung von 160 g nach c) hergestelltem Adipinsäuremonomethylester in 270 g Wasser wurden 16 g eines starksauren Ionenaustauschers (vernetztes Polystyrol mit Sulfonsäuregruppen) suspendiert. Das Reaktionsgemisch wurde in einem 1-l-Dreihalskolben mit aufgesetzter Drehbandkolonne so lange erhitzt, bis kein Methanol mehr überging. Der Ionenaustauscher wurde abfiltriert; aus dem Filtrat wurden nach Kristallisation und teilweisem Einengen der Mutterlauge 144,5 g Adipinsäure (Schmelzpunkt 153°C) erhalten (Ausbeute 99 %).

## Patentansprüche

1. Verfahren zur Herstellung von Adipinsäure, welches folgende Schritte umfaßt
a) Hydroformylierung von Pentensäureestern durch Umsetzung mit Kohlenmonoxid und Wasserstoff bei einer Temperatur von 60 bis 160°C und unter erhöhtem Druck in Gegenwart von Kobalt- oder Rhodiumcarbonylkomplexen unter Bildung eines Gemisches aus 5-, 4- und 3-Formylvaleriansäureestern
b) Abtrennen von 5-Formylvaleriansäureestern aus dem so erhaltenen Gemisch aus 5-, 4- und 3-Formylvaleriansäureestern unter Verbleib eines im wesentlichen aus 4- und 3-Formylvaleriansäureestern bestehenden Gemisches
c) Dehydrocarbonylieren des im wesentlichen aus 4- und 3-Formylvaleriansäureestern bestehenden Gemisches in Gegenwart von mindestens einem Element der 8. Nebengruppe des periodischen Systems bei einer Temperatur von 50 bis 400°C unter Bildung von Pentensäureestern und deren Rückführung in Stufe a) zur Hydroformylierung
d) Oxidieren der 5-Formylvaleriansäureester aus Stufe b) mit molekularem Sauerstoff oder solchen enthaltenden Gasen zu Adipinsäuremonoestern und
e) Hydrolysieren der Adipinsäuremonoester zu Adipinsäure.

2. Verfahren nach Anspruch 1, dadurch gekennzeichnet, daß man in Stufe a) eine Kobaltkonzentration von 0,05 bis 0,3 Mol.%, bezogen auf Pentensäureester einhält.

3. Verfahren nach den Ansprüchen 1 und 2, dadurch gekennzeichnet, daß man in der Stufe a) einen Umsatz an Pentensäureester von 10 bis 50 % einhält.

4. Verfahren nach den Ansprüchen 1 bis 3, dadurch gekennzeichnet, daß man in Stufe c) Trägerkatalysatoren verwendet, die 0,01 bis 10 Gew.% mindestens eines Metalls der 8. Nebengruppe des periodischen Systems enthalten.

5. Verfahren nach den Ansprüchen 1 bis 4, dadurch gekennzeichnet, daß man Trägerkatalysatoren verwendet, die mindestens 2 Edelmetalle der 8. Nebengruppe des periodischen Systems enthalten.

6. Verfahren nach den Ansprüchen 1 bis 4, dadurch gekennzeichnet, daß man Trägerkatalysatoren verwendet, die mindestens ein Edelmetall der 8. Nebengruppe des periodischen Systems und mindestens ein Metall, ausgewählt aus der Gruppe Eisen, Kobalt und Nickel enthalten.

7. Verfahren nach den Ansprüchen 1 bis 6, dadurch gekennzeichnet, daß man in der Stufe c) einen Katalysator verwendet, der zusätzlich mindestens eines der Elemente Kupfer, Silber, Zink, Titan, Vanadium, Chrom, Molybdän, Wolfram, Mangan oder Rhenium enthält.

8. Verfahren nach den Ansprüchen 1 bis 7, dadurch gekennzeichnet, daß man in der Stufe c) eine Temperatur von 60 bis 350°C einhält.

9. Verfahren nach den Ansprüchen 1 bis 8, dadurch gekennzeichnet, daß man in der Stufe c) molekularen Sauerstoff oder solchen enthaltende Gase mitverwendet.

10. Verfahren nach den Ansprüchen 1 bis 9, dadurch gekennzeichnet, daß man ein Molverhältnis von Formylvaleriansäureester zu molekularem Sauerstoff von 1:0,05 bis 1:3 einhält.

11. Verfahren nach den Ansprüchen 1 bis 3, dadurch gekennzeichnet, daß man in Stufe c) Verdünnungsmittel mitverwendet.

12. Verfahren nach den Ansprüchen 1 bis 11, dadurch gekennzeichnet, daß man die Umsetzung in Stufe c) unter Mitverwendung von Wasser oder Alkanolen mit 1 bis 6 Kohlenstoffen oder Gemischen derselben durchführt.

## Claims

1. A process for the preparation of adipic acid which comprises the following steps:
a) Hydroformylation of a pentenoic ester by reaction with carbon monoxide and hydrogen at from 60 to 160°C and under superatmospheric pressure in the presence of a cobalt carbonyl or rhodium carbonyl complex with formation of a mixture of 5-, 4- and 3-formylvaleric esters,
b) isolation of the 5-formylvaleric ester from the resulting mixture of 5-, 4- and 3-formylvaleric esters, a mixture essentially consisting of 4- and 3-formylvaleric esters remaining,
c) dehydrocarbonylation of the mixture consisting essentially of 4- and 3-formylvaleric esters in the presence of one or more elements of subgroup 8 of the Periodic Table at from 50 to 400°C with formation of pentenoic esters, and recycling of the latter to stage a) for hydroformylation,
d) oxidation of the 5-formylvaleric ester from stage b) with molecular oxygen or a gas containing molecular oxygen to give a monoester of adipic acid, and
e) hydrolysis of the monoester of adipic acid to give adipic acid.

2. A process as claimed in claim 1, wherein, in stage a), a cobalt concentration of from 0.05 to 0.3 mol %, based on pentenoic esters, is maintained.

3. A process as claimed in claim 1 or 2, wherein, in stage a), a conversion of pentenoic esters of from 10 to 50% is maintained.

4. A process as claimed in any of claims 1 to 3, wherein, in stage c), a supported catalyst is used which contains from 0.01 to 10% by weight of one or more metals of subgroup 8 of the Periodic Table.

5. A process as claimed in any of claims 1 to 4, wherein a supported catalyst is used which contains two or more noble metals of subgroup 8 of the Periodic Table.

6. A process as claimed in any of claims 1 to 4, wherein a supported catalyst is used which contains one or more noble metals of subgroup 8 of the Periodic Table and one or more metals selected from the group consisting of iron, cobalt and nickel.

7. A process as claimed in any of claims 1 to 6, wherein, in stage c), a catalyst is used which additionally contains one or more of the elements copper, silver, zinc, titanium, vanadium, chromium, molybdenum, tungsten, manganese and rhenium.

8. A process as claimed in any of claims 1 to 7, wherein, in stage c), a temperature of from 60 to 350°C is maintained.

9. A process as claimed in any of claims 1 to 8, wherein, in stage c), molecular oxygen or a gas containing molecular oxygen is concomitantly used.

10. A process as claimed in any of claims 1 to 9, wherein a molar ratio of formylvaleric esters to molecular oxygen of from 1 : 0.05 to 1 : 3 is maintained.

11. A process as claimed in any of claims 1 to 3, wherein, in stage c), a diluent is concomitantly used.

12. A process as claimed in any of claims 1 to 11, wherein the reaction in stage c) is carried out in the presence of water or of an alkanol of 1 to 6 carbon atoms or of a mixture of these.

## Revendications

1. Procédé de préparation d'acide adipique, comprenant les étapes suivantes:
a) Hydroformylation d'esters d'acide penténoïque par réaction avec du monoxyde de carbone et de l'hydrogène à une température de 60 à 160°C et sous pression élevée en présence de complexes cobalt- ou rhodiumcarbonyle, avec formation d'un mélange d'esters d'acides 5-, 4- et 3-formylvalériques,
b) Séparation des esters d'acide 5-formylvalérique d'avec le mélange d'esters d'acides 5-, 4- et 3-formylvalériques ainsi obtenu, de sorte qu'il reste un mélange se composant essentiellement d'esters d'acides 4- et -3-formylvalériques,
c) Déshydrocarbonylation du mélange composé essentiellement d'esters d'acides 4- et 3-formylvalériques en présence d'au moins un élément du groupe VIII du système périodique, à une température de 50 à 400°C, avec formation d'esters d'acide penténoïque, et recyclage de ceux-ci dans l'étape a) d'hydroformylation,
d) Oxydation des esters d'acide 5-formylvalérique provenant de l'étape b) avec de l'oxygène moléculaire ou des gaz contenant celui-ci, pour l'obtention de monoesters d'acide adipique, et
e) Hydrolyse des monoesters d'acide adipique en acide adipique.

2. Procédé selon la revendication 1, caractérisé en ce qu'on maintient, dans l'étape a), une concentration de cobalt de 0,05 à 0,3% en moles, par rapport aux esters d'acide penténoïque,

3. Procédé selon la revendication 1 ou 2, caractérisé en ce qu'on maintient, dans l'étape a), un degré de transformation des esters d'acide penténoïque de 10 à 50%.

4. Procédé selon l'une quelconque des revendications 1 à 3, caractérisé en ce qu'on utilise, dans l'étape c), des catalyseurs sur support qui contiennent de 0,01 à 10% en poids d'au moins un métal du groupe VIII du système périodique.

5. Procédé selon l'une quelconque des revendications 1 à 4, caractérisé en ce qu'on utilise des catalyseurs sur support qui contiennent au moins 2 métaux précieux du groupe VIII du système périodique.

6. Procédé selon l'une quelconque des revendications 1 à 4, caractérise en ce qu'on utilise des catalyseurs sur support qui contiennent au moins un métal précieux du groupe VIII du système périodique et au moins un métal choisi dans le groupe du fer, du cobalt et du nickel.

7. Procédé selon l'une quelconque des revendications 1 a 6, caractérisé en ce qu'on utilise, dans l'étape c), un catalyseur qui contient en plus au moins l'un des éléments cuivre, argent, zinc, titane, vanadium, chrome, molybdène, tungstène, manganèse et rhénium.

8. Procédé selon l'une quelconque des revendications 1 a 7, caractérisé en ce qu'on maintient, dans l'étape c), une température de 60 à 350°C.

9. Procédé selon l'une quelconque des revendications 1 à 8, caractérisé en ce qu'on utilise simultanément, dans l'étape c), de l'oxygène moléculaire ou des gaz contenant celui-ci.

10. Procédé selon l'une quelconque des revendications 1 à 9, caractérisé en ce qu'on maintient un rapport molaire des esters d'acides formylvalériques à l'oxygène moléculaire de 1:0,05 à 1:3.

11. Procédé selon l'une quelconque des revendications 1 à 3, caractérisé en ce qu'on utilise simultanément, dans l'étape c), des diluants.

12. Procédé selon l'une quelconque des revendications 1 à 11, caractérisé en ce qu'on conduit la réaction, dans l'étape c), en utilisant simultanément de l'eau, des alcanols à 1-6 atomes de carbone ou des mélanges de ceux-ci.
